Europäisches Patentamt

European Patent Office

Office européen des brevets

(11) Publication number: **0 298 527**
**A1**

## (12) EUROPEAN PATENT APPLICATION

(21) Application number: 88200890.7

(22) Date of filing: 04.05.88

(51) Int. Cl.⁴: **A61F 5/46 , A61K 9/02**

The title of the invention has been amended (Guidelines for Examination in the EPO, A-III, 7.3).

(30) Priority: 14.05.87 NL 8701160

(43) Date of publication of application:
**11.01.89 Bulletin 89/02**

(84) Designated Contracting States:
**AT BE CH DE ES FR GB GR IT LI LU NL SE**

(71) Applicant: **Fundatech S.A.**
**1,rue Robert-de-Traz Case postale 29**
**Champel**
**CH-1211 Genève - 12(CH)**

(72) Inventor: **Scouvart, Jean André Henri**
**Tir Aux Pigeons 72**
**B-1150 Brussels(BE)**

(74) Representative: **Kupecz, Arpad et al**
**Octrooibureau Los en Stigter B.V. Postbox**
**20052**
**NL-1000 HB Amsterdam(NL)**

(54) **Contraceptive suppository based on gelatine, and process for its manufacture.**

(57) The invention relates to a contraceptive suppository on gelatine base, which dissolves or melts under the influence of the circumstances present in the vagina, and to a process for the manufacture of the same.

The contraceptive suppository of the invention consists of a flat flexible disc and is composed of a mixture of glycerine and gelatine in a suitable proportion.

The contraceptive suppository contains preferably one or more pharmacologically active substances, such as nonoxynol-9 and/or an anti aids agent.

EP 0 298 527 A1

## Contraceptive suppository on gelatine base and process for the manufacture thereof.

The invention relates to a contraceptive suppository on gelatine base, as well as to a process for the manufacture thereof.

Such a contraceptive suppository and a process for the manufacture thereof are known from Dutch patent specification Nr. 123030. The contraceptive suppository according to this patent specification consists of a slightly solid cup, which in shape and dimensions has been made suitable to partly cover the top portion of the penis. The cup of the known contraceptive suppository comprises on the inner side in the centre a flat or almost flat portion, which is perpendicular to the axis of the cup and at least two ribs extend from this flat portion to the outer edge to the cup. Moreover a small amount of an agglutinant has been applied on the central flat portion, which works temporarily as an adhesive.

The function of the adhesive is that the contact surface with the top portion of the penis is small and on the other hand that the adhesion on insertion is satisfactory. This adhesion decreases rapidly and becomes so slight, that the suppository stays behind in the vagina with certainty.

The known contraceptive suppository has a complicated construction, with the additional disadvantage, that herewith use is to be made of an adhesive. Moreover the cup is intended to be placed on the top portion of the penis.

It is an object of the present invention to provide a simpler contraceptive suppository, which may be easily inserted in the vagina with the finger or with another suitable apparatus prior to intercourse.

The contraceptive suppository according to the invention is characterized in that it consists of a flat flexible disc and is composed of a mixture of glycerine and gelatine in a suitable proportion.

The new contraceptive suppository is usually circular, but of course it may have another shape, for example oval.

A particular advantage of the new contraceptive suppository is that it is very flexible, to such extent that after having been inserted in the vagina no mechanical irritation is caused, so that bleedings if any do not take place. Also because of its high degree of flexibility this material has the advantage, that during intercourse neither the woman, nor the man experiences any inconvenience.

The contraceptive suppository inserted prior to intercourse is dissolved in the vagina or it melts within about three hours.

The solubility of the melting capacity of the contraceptive suppository according to the invention may be controlled by attuning the proportion of glycerine and gelatine to each other.

As gelatine usually gelatine "Bloom Grade 240" is used, whereas as glycerine the usual glycerine which is commercially available is used.

Preferably the contraceptive suppository contains a pharmacologically active substance, in particular a spermicide, such as nonoxynol-9. When using nonoxynol-9, gelatine has the advantage that the dryness and irritation usually caused by nonoxynol-9 do not occur. Nonoxynol-9 namely is a soapy substance.

Beside or instead of nonoxynol-9 other pharmacologically acceptable active substances may be used, among which an anti aids agent.

Since the contraceptive suppository according to the invention gradually dissolves or melts within about three hours, the active substances are gradually released in order to adequately effect the spermicide activity and/or other pharmacological activity.

It is observed that beside the said spermicides and anti aids agent of course other substances may be incorporated in the suppository, such as antibiotics, fungicides etc.

It stands to reason that an effective quantity of the active substance is used, which quantity varies within wide limits, dependent on for instance the seriousness of the infection. This effective quantity of active substance in the contraceptive suppository lies between 1 - 11%, by weight.

The invention furthermore relates to a process for the manufacture of a contraceptive suppository, characterized in that glycerine is dissolved in water in a suitable quantity at raised temperature, whereafter a suitable quantity of gelatine is added to the thus obtained solution and the raised temperature is maintained until the gelatine is molten, followed by pouring out the thus obtained mixture on a glass plate or the like and cooling to room temperature.

The contraceptive suppository obtained after cooling may be vacuum packed ready to use and as such may be put on the market.

In order to decrease the desired activity of the contraceptive suppository one or more pharmacologically active substances are added to the mixture of glycerine and gelatine and are homogeneously admixed therein. As spermicide preferably nonoxynol-9 is used in a quantity of 1 - 11%, by weight. Furthermore as active substance an anti aids agent may be succesfully incorporated in the contraceptive suppository.

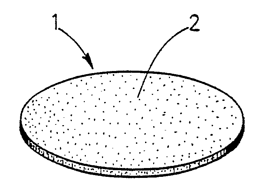

The contraceptive suppository according to the invention will now be further elucidated with reference to the drawing.

The figure shows a perspective view of the contraceptive suppository according to the invention.

The disc shaped contraceptive suppository 1 according to the invention is circular or oval, in which the pharmacologically active substances 2 are homogeneously divided.

The thickness of the suppository may vary from 1 - 4 mm. The diameter may vary from 3 - 7 cm.

It is observed that in the contraceptive suppository the quantity of active substance or substances is between 1 - 11%, by weight.

The process according to the invention is further elucidated with reference to the following example.

Example

22 g water and 60 g glycerine were mixed with each other and subsequently heated in a water bath to 80° C until all glycerine was dissolved. 18 g gelatine was added to the thus obtained solution while stirring slowly, whereas the mixture was maintained at approximately 80° C in the same water bath. The heating was continued until the gelatine was molten.

While stirring 11 g nonoxynol-9 was added to the molten mixture and was thoroughly mixed therewith. After obtaining a homogeneous melt this was poured out on a petri dish having a diameter of approximately 5,5 cm. After cooling at room temperature in the air a flexible to the touch and flat contraceptive suppository was obtained, which was thereafter ready to be vacuum packed and to be put on the market as such.

This contraceptive suppository has a thickness of approximately 2 mm and a diameter of approximately 5,5 cm. In an analogous way contraceptive suppositories may be manufactured having thicknesses varying from 1 - 4 mm and having diameters varying from 1 - 7 cm.

Claims

1. Contraceptive suppository on gelatine base, which dissolves or melts under the influence of the circumstances present in the vagina, characterized in that the contraceptive suppository consists of a flat flexible disc and is composed of a mixture of glycerine and gelatine in a suitable proportion.

2. Contraceptive suppository according to claim 1, characterized in that the proportion of glycerine to gelatine is approximately 3:1.

3. Contraceptive suppository according to claim 1 or 2, characterized in that it contains one or more pharmacologically active substances.

4. Contraceptive suppository according to claim 3, characterized in that the active substance is nonoxynol-9.

5. Contraceptive suppository according to claim 3, characterized in that the active substance is an anti aids agent.

6. Contraceptive suppository according to claims 1 - 5, characterized in that the contents of the active substance is 1 - 11%, by weight.

7. Process for the manufacture of a contraceptive suppository according to claims 1 - 6, characterized in that glycerine is dissolved in water in a suitable quantity at raised temperature, whereafter a suitable quantity of gelatine is added to the thus obtained solution and the raised temperature is maintained until the gelatine is molten, followed by pouring out the thus obtained mixture on a glass plate or the like and cooling to room temperature.

8. Process according to claim 7, characterized in that one or more pharmacologically active substances are added to the mixture of glycerine and gelatine and are homogeneously admixed.

9. Process according to claim 7 or 8, characterized in that as pharmacologically active substance nonoxynol-9 is used in a quantity of 1 - 11%, by weight.

10. Process according to claim 7 or 8, character ized in that as pharmacologically active substance an anti aids agent is used.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | NL-A-6 902 076 (EISAI K.K.)<br>* Claim 1 *<br>--- | 1-10 | A 61 F  5/46<br>A 61 K  9/02 |
| Y | FR-A-1 563 734 (CHEMOLIMPEX MAGYAR VEGYIARU KÜLKERESKEDELMI VALLALAT)<br>* Page 2, left-hand column, lines 25-32; page 2, right-hand column, lines 15-24; page 3, left-hand column, lines 4-6 *<br>--- | 1-10 | |
| Y | US-A-4 187 286 (MARCUS)<br>* Column 1, lines 56-62 *<br>--- | 4-6,9, 10 | |
| Y | DE-A- 832 045 (LEHNERT)<br>* Claims 13-17 *<br>--- | 7,8 | |
| Y | DE-A- 883 190 (RÜTHER et al.)<br>* Page 2, lines 10-24,37-39 *<br>--- | 1 | |
| Y | NL-A- 123 030 (ETABLISSEMENT TA-RUX)<br>* Column 2, lines 15-20 *<br>----- | 1 | TECHNICAL FIELDS SEARCHED (Int. Cl.4)<br><br>A 61 F<br>A 61 K |

The present search report has been drawn up for all claims

| Place of search | Date of completion of the search | Examiner |
|---|---|---|
| THE HAGUE | 22-08-1988 | GLAS J. |